# EUROPÄISCHE PATENTSCHRIFT

(11) **EP 0 803 488 B1**
(45) Veröffentlichungstag und Bekanntmachung des Hinweises auf die Patenterteilung: **27.03.2002**
(21) Anmeldenummer: 97106904.2
(22) Anmeldetag: 25.04.1997
(51) Int. Cl.: C07B 35/02, C07C 29/136, C07C 29/19, C07C 31/12, C07C 31/135, C07C 31/20, C07C 35/08, C07C 209/72, C07C 211/35, C08F 8/04

(54) **Verfahren zur Umsetzung einer organischen Verbindung in Gegenwart eines in situ gebildeten Ruthenium-katalysators**
Process for reacting an organic compound in the presence of a ruthenium catalyst formed in situ
Procédé pour la réaction d'un compose organique en présence d'un catalyseur au ruthénium formé in situ

(30) Priorität: 26.04.1996 DE 19616822; 05.06.1996 DE 19622705
(43) Veröffentlichungstag der Anmeldung: 29.10.1997
(73) Patentinhaber: BASF AKTIENGESELLSCHAFT, 67056 Ludwigshafen (DE)
(72) Erfinder: Rühl, Thomas Dr., 67227 Frankenthal (DE); Breitscheidel, Boris, Dr., 67117 Limburgerhof (DE); Bröcker, Franz-Josef, Dr., 67061 Ludwigshafen (DE); Reif, Wolfgang, Dr., 67227 Frankenthal (DE); Henkelmann, Jochem, Dr., 68165 Mannheim (DE); Brauch, Karl Heinz, 68623 Lampertheim (DE); Henne, Andreas, Dr., 67433 Neustadt (DE)
(74) Vertreter: Isenbruck, Günter, Dr.

(56) Entgegenhaltungen:
- EP-A- 0 141 054
- EP-A- 0 501 265
- DE-A- 2 132 547
- DE-A- 2 909 663
- US-A- 4 464 482
- US-A- 4 749 677

## Beschreibung

Die vorliegende Erfindung betrifft ein Verfahren zur Durchführung einer Katalytischen Umsetzung einer organischen Verbindung in Gegenwart eines Katalysators, wobei der Katalysator eine auf einem Träger aufgebrachte Rutheniumverbindung umfaßt, dadurch gekennzeichnet, daß der katalysator während der katalytischen Umsetzung der organischen Verbindung durch das Abschneiden einer homogenen Rutheniumverbindung auf dem Träger gebildet wird.

In einer Ausführungsform betrifft die vorliegende Erfindung ein Verfahren zur Hydrierung einer aromatischen Verbindung, in der mindestens eine Hydroxylgruppe an einen aromatischen Kern gebunden ist, wobei vorzugsweise zusätzlich zur mindestens einen Hydroxylgruppe mindestens eine, gegebenenfalls substituierte, C₁₋₁₀-Alkylgruppe und/oder mindestens eine C₁₋₁₀-Alkoxygruppe an einen aromatischen Kern gebunden ist. Des weiteren werden Monoalkyl-substituierte Phenole im erfindungsgemäßen Verfahren bevorzugt eingesetzt. Die ein- oder mehrkernigen aromatischen Verbindungen werden dabei in Gegenwart des oben beschriebenen Katalysators zu den entsprechenden cycloaliphatischen Verbindungen hydriert, wobei die Hydroxylgruppe erhalten bleibt.

Cycloaliphatische Alkohole, insbesondere Alkylcyclohexanole, sind wichtige Zwischenprodukte für die Herstellung verschiedener Duftstoffe, Arzneimittel und anderer organischer Feinchemikalien. Durch katalytische Hydrierung der entsprechenden aromatischen Vorläufer sind die obigen cycloaliphatischen Alkohole bequem zugänglich.

Das Verfahren, Alkylcyclohexanole durch katalytische Hydrierung der entsprechenden Alkylphenole herzustellen, ist bekannt. Die Hydrierung von Alkylphenolen zu den entsprechenden Alkylcyclohexanolen in Gegenwart von Hydrierungskatalysatoren, insbesondere auf Trägern aufgebrachten Katalysatoren, ist vielfach beschrieben.

Als Katalysatoren werden beispielsweise metallisches Rhodium, Rhuthenium, Palladium sowie Nickel auf Katalysatorträgern verwendet. Als Katalysatorträger werden Kohlenstoff, Bariumcarbonat und insbesondere Aluminiumoxid eingesetzt.

In der PL 137 526 ist die Hydrierung von p-tert.-Butylphenol zu p-tert.-Butylcyclohexanol unter Verwendung eines Nickel-Katalysators beschrieben.

In der DE-A-34 01 343 und der EP 0 141 054 ist ein Verfahren zur Herstellung von 2- und 4-tert.-Butylcyclohexanol aus 2- und 4-tert.-Butylphenol durch katalytische Hydrierung beschrieben. Die Hydrierung wird zweistufig ausgeführt, wobei in der ersten Stufe ein Palladium-Katalysator auf einem Al₂O₃-Träger und in der zweiten Stufe ein Ruthenium-Katalysator auf einem Al₂O₃-Träger verwendet wird. Der Metallgehalt auf dem Träger beträgt dabei 0,1 bis 5 Gew.-%. Die Träger sind nicht weiter spezifiziert. Es wird bei einem Druck von 300 bar unter Produktrückführung gearbeitet, und es werden vorzugsweise die cis-tert.-Butylphenole erhalten, wobei 0,1 bis 0,5 % Nebenprodukte anfallen.

Die US 2,927,127 beschreibt ein Verfahren zur Herstellung von p-tert.-Butylcyclohexanol und Estern davon durch katalytische Hydrierung von p-tert.-Butylphenol. Als Katalysatoren werden 5 % Rhodium auf Kohlenstoff, 5 % Palladium auf Bariumcarbonat und 5 % Ruthenium auf Kohlenstoff verwendet. Bei der Verwendung von Ruthenium auf Kohlenstoff wurde mit einem Druck von 70 bis 120 bar und einer Temperatur von 74 bis 93 °C gearbeitet. Als Hydrierungsprodukt wurden 66% cis-Isomer erhalten.

In der DE-A-29 09 663 ist ein Verfahren zur Herstellung von cis-Alkylcyclohexanolen durch katalytische Hydrierung der entsprechenden Alkylphenole beschrieben. Als Katalysator wurde Ruthenium auf einem Al₂O₃-Träger verwendet. Es wurde bei Drücken von 40, 60 oder 80 bar gearbeitet. Als Produkt wurden überwiegend cis-Alkylcyclohexanole erhalten, wobei als Nebenprodukt 0,1 bis 1 % Alkylbenzole anfielen.

Weiterhin beschreibt die US-PS-4 749 677 ein Verfahren zur Herstellung einer Katalysatorzusammensetzung, die aus einer Aufschlämmung besteht, wobei als Trägermaterial Titandioxid eingesetzt wird, welches mit einem Rutheniumcarbonylkomplex in einem inerten Kohlenwasserstoff umgesetzt wurde. Dieser Katalysator wird anschließend in einer Abfolge von drei Reaktionsschritten, nämlich das Hinzufügen von Rutheniumcarbonylkomplex in einem inerten Alkankohlenwasserstoff zu einem aufgeschlämmten TiO₂, das Hinzufügen einer Gasmischung aus Stickstoff und Kohlenmonoxid zu der Aufschlämmung, und weiterem hinzufügen einer Gasmischung aus Stickstoff und Wasserstoff zur erwähnter Aufschlämmung und gleichzeitigem Erhöhen der Temperatur von 20 auf 250°C und gleichzeitigem Erhöhen des Druckes von 1 bis ungefähr 20 Atmospheren über einen Zeitraum von 2 bis 6 Stunden, hergestellt. Erst nach Herstellung des vorerwähnten Katalysators kann eine katalytische Reaktion durchgeführt werden. Der dergestalt hergestellte Katalysator wird zur Herstellung von Kohlenwasserstoffen aus Kohlenmonoxid und Wasserstoff in der sogenannten "Fischer-Tropsch-Synthese" eingesetzt. Vorerwähntes US-Patent schweigt sich jedoch über den Einsatz von organischen Materialien zur Durchführung einer katalytischen Umsetzung aus.

In einer weiteren Ausführungsform betrifft die vorliegende Erfindung ein Verfahren zur Hydrierung einer aromatischen Verbindung, in der mindestens eine Aminogruppe an einen aromatischen Kern gebunden ist, wobei vorzugsweise zusätzlich zur mindestens einen Aminogruppe mindestens eine, ggf. substituierte, C₁₋₁₀-Alkylgruppe und/oder mindestens eine C₁₋₁₀-Alkoxygruppe an einen aromatischen Kern gebunden ist. Insbesondere bevorzugt werden Monoallyl-substituierte Amine eingesetzt.

Die ein- oder mehrkernigen aromatischen Verbindungen werden dabei in Gegenwart des eingangs beschriebenen Katalysators zu den entsprechenden cycloaliphatischen Verbindungen hydriert, wobei die Aminogruppe erhalten bleibt.

Cycloaliphatische Amine, insbesondere gegebenenfalls substituierte Cyclohexylamine und Dicyclohexylamine, finden Verwendung zur Herstellung von Alterungsschutzmitteln für Kautschuke und Kunststoffe, als Korrosionsschutzmittel sowie als Vorprodukte für Pflanzenschutzmittel und Textilhilfsmittel. Cycloaliphatische Diamine werden zudem bei der Herstellung von Polyamid- und Polyurethanharzen eingesetzt und finden weiterhin Verwendung als Härter für Epoxidharze.

Es ist bekannt, cycloaliphatische Amine durch katalytische Hydrierung der entsprechenden ein- oder mehrkernigen aromatischen Amine herzustellen. Die Hydrierung von aromatischen Aminen zu den entsprechenden cycloaliphatischen Aminen in Gegenwart von Hydrierungskatalysatoren, insbesondere auf Trägern aufgebrachten Katalysatoren, ist vielfach beschrieben.

Als Katalysatoren werden beispielsweise Raney-Kobalt mit basischen Zusätzen (JP 43/3180), Nickel-Katalysatoren (US 4,914,239, DE-A 80 55 18), Rhodium-Katalysatoren (BE 73 93 76, JP 70 19 901, JP 72 35 424), sowie Palladiumkatalysatoren (US 3,520,928, EP-A 0 501 265, EP-A 0 053 818, JP 59/196 843) verwendet. In der Mehrzahl werden jedoch rutheniumhaltige Katalysatoren eingesetzt.

Aus der DE-A 21 32 547 ist ein Verfahren zur Hydrierung ein- oder mehrkerniger aromatischer Diamine zu den entsprechenden cycloaliphatischen Aminen bekannt, das in Gegenwart eines suspendierten Rutheniumkatalysators ausgeführt wird.

In der EP-A 0 067 058 ist ein Verfahren zur Herstellung von Cyclohexylamin durch katalytische Hydrierung des entsprechenden aromatischen Amins beschrieben. Als Katalysator wird Rutheniummetall in einer fein verteilten Form auf aktivierten Aluminiumpellets verwendet. Nach vier Rückführungen begann der Katalysator seine Wirksamkeit zu verlieren.

Die EP-A 0 324 984 betrifft ein Verfahren zur Herstellung eines Gemisches aus gegebenenfalls substituiertem Cyclohexylamin und gegebenenfalls substituiertem Dicyclohexylamin durch Hydrierung von gegebenenfalls substituiertem Anilin unter Verwendung eines Ruthenium und Palladium auf einem Träger enthaltenden Katalysators, der darüber hinaus eine alkalisch reagierende Alkalimetallverbindung als Modifiziermittel enthält. Ein prinzipiell ähnliches Verfahren ist in der EP-A 0 501 265 beschrieben, wobei dort der Katalysator als Modifiziermittel Niobsäure, Tantalsäure oder ein Gemisch beider enthält.

In der US 2,606,925 wird ein Verfahren zur Herstellung einer Aminocyclohexyl-Verbindung durch Hydrieren einer entsprechenden aromatischen Verbindung beschrieben, wobei ein Rutheniumkatalysator, dessen aktive katalytische Komponente ausgewählt wird unter elementarem Ruthenium, Rutheniumoxiden, Rutheniumsalzen, in denen das Ruthenium im Anion oder im Kation vorhanden ist. Wie die Beispiele dieses Verfahrens zeigen, wird auch dort der Katalysator in einer getrennten Stufe hergestellt und getrocknet und nach längerer Trocknungszeit in das Reaktionsgefäß eingebracht.

Ein weiteres Verfahren zur Herstellung von Cyclohexylamin wird in der US 2,822,392 beschrieben, wobei das Hauptaugenmerk dieser Patentschrift auf die Verwendung eines speziellen Reaktors, in dem das Anilin und der Wasserstoff als Ausgangsprodukte im Gegenstrom miteinander zur Umsetzung gebracht werden, gerichtet ist.

Die US 3,636,108 und US 3,697,449 betreffen die katalytische Hydrierung aromatischer, Stickstoff enthaltender Verbindung unter Verwendung eines Rutheniumkatalysators, der zusätzlich eine Alkalimetallverbindung als Modifiziermittel umfaßt.

Ferner betrifft die vorliegende Erfindung insbesondere ein Verfahren zur Hydrierung einer organischen Verbindung, die mindestens eine C=O-Gruppe aufweist, wie z. B. ein Keton, Aldehyd, eine Carbonsäure oder ein Derivat davon, oder ein Gemisch aus zwei oder mehr davon.

Die US 4,464,482 beschreibt einen Ruthenium-Katalysator für Hydrierungen, der aus einem Metallträger mit spezieller Struktur, einem darauf abgeschiedenen Überzug aus aktiviertem Aluminiumoxid und einer auf diesem Überzug aufgetränkte Rutheniummenge von 0,03 bis 3 Gew.-% besteht. Die Oxidschicht wird durch Tauchen des Trägers in eine Dispersion von aktiviertem Aluminiumoxid und anschließender Temperung oder - sofern der Metallträger Aluminium enthält - durch Temperung desselben erzeugt. Ferner wird die Verwendung dieses Katalysators für eine Reihe von Hydrierungsreaktionen von insbesondere Aldehyden und Ketonen beschrieben.

Wie sich aus obiger Zusammenfassung des Standes der Technik ergibt, wurde innerhalb der oben beschriebenen Verfahren der verwendete Rutheniumkatalysator immer in einem separaten Schritt hergestellt. Dabei wird die Aktivkomponente auf den Katalysatorträger aufgetränkt oder aufgespritzt und nach einem Trocknungsschritt bei hohen Temperaturen kalziniert. Anschließend wird in den meisten Fällen der Katalysator in einem Wasserstoffstrom bei erhöhter Temperatur aktiviert.

Ein Aufgabe der vorliegenden Erfindung lag demgemäß in der Bereitstellung eines Verfahrens zur Durchführung einer Katalytischen Umsetzung einer organischen Verbindung, wie eingangs definiert, wobei sehr hohe Ausbeuten beziehungsweise nahezu vollständiger Umsatz erreicht werden.

Eine weitere Aufgabe der Erfindung liegt in der Bereitstellung eines derartigen Verfahrens, wobei lediglich ein minimaler Anteil von Nebenprodukten beziehungsweise Zersetzungsprodukten während der Hydrierung anfällt.

Weiterhin sollte es möglich sein, das erfindungsgemäße Verfahren ohne Katalysatorabtrennung, -aufarbeitung und -rückführung durchzuführen.

Ferner sollte es möglich sein, das Verfahren unter hohen Katalysatorbelastungen und langen Standzeiten mit einer extrem hohen Turn-Over-Zahl durchzuführen, wobei die entsprechenden Hydrierungsprodukte in hoher Ausbeute und hoher Reinheit erhalten werden.

Gelöst werden eine oder mehrere der vorstehenden Aufgaben durch das eingangs erwähnte Verfahren zur Durchführung einer Katalytischen Umsetzung einer organischen Verbindung, in Gegenwart eines Katalysators einschließlich niedermolekularer (monomerer) und polymerer organischer Verbindungen (Polymere).

Ein besonderer Vorteil der Erfindung ist darin zu sehen, daß der Reaktionsaustrag frei von Rutheniumspuren ohne Katalysatorabtrennung direkt weiter verwendet werden kann.

Ferner weist das erfindungsgemäße Verfahren hohe Turn-Over-Zahlen bei hoher Katalysatorbelastung und über lange Katalysatorstandzeiten hinweg auf. Die Katalysatorbelastung ist dabei die Raum/Zeit-Ausbeute des Verfahrens, d.h. die Menge des umgesetzten Edukts pro Zeiteinheit und pro Menge an vorliegendem Katalysator. Standzeit bedeutet die Zeit bzw. die Menge an umgesetztem Edukt, die ein Katalysator verkraftet, ohne seine Eigenschaften einzubüßen und ohne daß sich die Produkteigenschaften signifikant verändern. Dabei werden bei dem hierin beschriebenen Verfahren die entsprechenden Umsetzungsprodukte in hoher Ausbeute und Reinheit erhalten.

Weiterhin wurde gefunden, daß es mit den erfindungsgemäß verwendeten Katalysatoren möglich ist, Flüssigkeitsbelastungen von >300 m³/m² ohne mechanische Beeinträchtigung einzustellen. Damit können bei optimalen Stoffübergang Umsetzungen, vorzugsweise Hydrierungen mit sehr hoher Wärmetönung sicher gehandhabt werden.

### VERBINDUNGEN

Der Begriff "organische Verbindung", wie er im Rahmen der vorliegenden Erfindung verwendet wird, umfaßt alle organischen Verbindungen, die katalytisch umgesetzt werden können, insbesondere solche, die mit Wasserstoff zu behandelnde Gruppen, wie z.B. C-C-Doppel- oder Dreifach-Bindungen, aufweisen.

Die vorliegende Erfindung betrifft insbesondere ein Verfahren zur Umsetzung einer organischen Verbindung in Gegenwart eines Katalysators, wobei die Umsetzung eine Hydrierung, Dehydrierung, Hydrogenolyse, aminierende Hydrierung oder Dehalogenierung, weiter bevorzugt eine Hydrierung ist.

Dabei können insbesondere organische Verbindungen eingesetzt werden, die eine oder mehrere der folgenden Struktureinheiten aufweisen:

C=C (I)

C≡C (II)

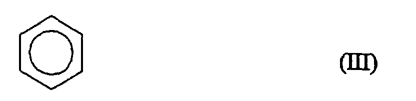

C=N (IV)

C≡N (V)

C=O (VI)

C=S (VII)

―NO₂ (VIII)

Besonders geeignet ist das erfindungsgemäße Verfahren zur Umsetzung, vorzugsweise Hydrierung, einer organischen Verbindung, die ausgewählt wird aus der Gruppe bestehend aus einer aromatischen Verbindung, in der mindestens eine Hydroxylgruppe an einen aromatischen Kern gebunden ist, einer aromatischen Verbindung, in der mindestens eine Aminogruppe an einen aromatischen Kern gebunden ist, einem Keton, einem Aldehyd, einer Carbonsäure oder einem Derivat davon, einem Polymer, das mindestens eine C-C-Doppelbindung aufweist, einem Polymer, das mindestens eine C=O-Gruppe aufweist, und einem Gemisch aus zwei oder mehr davon.

Im Rahmen des erfindungsgemäßen Verfahrens können auch organische Verbindungen umgesetzt werden, die Einheiten verschiedener Strukturen, wie oben definiert, enthalten, z.B. organische Verbindungen, die sowohl C-C-Mehrfachbindungen als auch Carbonylgruppen aufweisen, da die im Rahmen des erfindungsgemäßen Verfahrens verwendeten Katalysatoren in der Lage sind, zunächst eine der beiden Gruppen selektiv umzusetzen, vorzugsweise zu hydrieren, d.h. eine Hydrierung dieser Gruppen von ungefähr 90 bis 100% zu erreichen, während zunächst gleichzeitig die anderen Gruppen zu weniger als 25% und im allgemeinen in einem Bereich von 0 bis ungefähr 7% umgesetzt, vorzugsweise hydriert werden. Dabei werden im allgemeinen zunächst die C-C-Mehrfachbindungen und anschließend die C=O-Gruppen umgesetzt bzw. hydriert.

Der Begriff "aromatische Verbindung, in der mindestens eine Hydroxylgruppe an einem aromatischen Kern gebunden ist" bzw. "aromatische Verbindung, in der mindestens eine Aminogruppe an einen aromatischen Kern gebunden ist" steht für alle Verbindungen, die eine Einheit der folgenden Struktur (I) aufweisen: 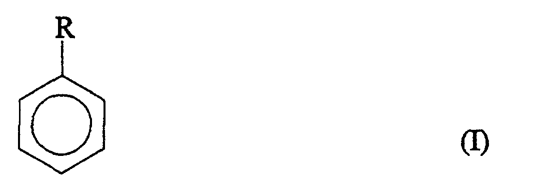 wobei R eine Hydroxyl- oder eine Aminogruppe darstellt.

Sofern im Rahmen des erfindungsgemäßen Verfahrens aromatische Verbindungen eingesetzt werden, in denen mindestens eine Hydroxylgruppe und ferner mindestens ein gegebenenfalls substituierter C₁₋₁₀-Alkylrest und/oder -Alkoxyrest an einen aromatischen Kern gebunden ist, kann je nach Reaktionsbedingungen (Temperatur, Lösungsmittel) das erhaltene Isomerenverhältnis von cis- zu trans-konfigurierten Produkten in einem weiten Bereich variiert werden. Ferner können die erhaltenen Verbindungen ohne weitere Reinigungsschritte weiterverarbeitet werden. Dabei wird die Bildung von Alkylbenzolen praktisch vollständig vermieden.

Wie auch die oben beschriebenen Verbindungen, in denen mindestens eine Hydroxylgruppe an einen aromatischen Kern gebunden ist, können im Rahmen des erfindungsgemäßen Verfahrens auch aromatische Verbindungen, in denen mindestens eine Aminogruppe an einen aromatischen Kern gebunden ist, mit hoher Selektivität zu den entsprechenden cycloaliphatischen Verbindungen hydriert werden, wobei für die zusätzlich mit einem C₁₋₁₀-Alkylrest und/oder -Alkoxyrest substituierten Amine bezüglich des Verhältnisses der cis- und trans-Isomeren das oben Gesagte gilt.

Insbesondere wird im Rahmen dieser Ausführungsform die Bildung von Desaminierungsprodukten, wie beispielsweise Cyclohexanen oder teilhydrierten Dimerisierungsprodukten wie Phenylcyclohexylaminen, praktisch vollständig vermieden.

Im einzelnen können im Rahmen des erfindungsgemäßen Verfahrens folgende Verbindungen umgesetzt werden:

### Aromatische Verbindungen, in denen mindestens eine Hydroxylgruppe an einen aromatischen Kern gebunden ist

Mit dem erfindungsgemäßen Verfahren können aromatische Verbindungen, in denen mindestens eine Hydroxylgruppe und vorzugsweise ferner mindestens ein gegebenenfalls substituierter C₁₋₁₀-Alkylrest und/oder Alkoxyrest an einen aromatischen Kern gebunden ist, zu den entsprechenden cycloaliphatischen Verbindungen hydriert werden, wobei auch Gemische zweier oder mehr dieser Verbindungen eingesetzt werden können. Dabei können die aromatischen Verbindungen einkernige oder mehrkernige aromatische Verbindungen sein. Die aromatischen Verbindungen enthalten mindestens eine Hydroxylgruppe, die an einen aromatischen Kern gebunden ist, die einfachste Verbindung dieser Gruppe ist Phenol. Vorzugsweise weisen die aromatischen Verbindungen eine Hydroxylgruppe pro aromatischem Kern auf. Die aromatischen Verbindungen können an dem aromatischen Kern oder den aromatischen Kernen substituiert sein durch einen oder mehrere Alkyl- und/oder Alkoxyreste, vorzugsweise C₁₋₁₀-Alkyl- und/oder Alkoxyreste, besonders bevorzugt C₁₋₁₀-Alkylreste, insbesondere Methyl-, Ethyl-, Propyl-, Isopropyl-, Butyl-, Isobutyl-, tert.-Butylreste; unter den Alkoxyresten sind die C₁₋₈-Alkoxyreste, wie z.B. Methoxy-, Ethoxy-, Propoxy-, Isopropoxy-, Butoxy-, Isobutoxy-, tert.-Butoxy-Reste, bevorzugt. Der aromatische Kern oder die aromatischen Kerne sowie die Alkyl- und Alkoxyreste können gegebenenfalls durch Halogenatome, insbesondere Fluoratome, substituiert sein oder andere geeignete inerte Substituenten aufweisen.

Vorzugsweise weisen die erfindungsgemäß hydrierbaren Verbindungen mindestens einen, vorzugsweise einen bis vier, insbesondere einen C₁₋₁₀-Alkylrest auf, der sich vorzugsweise am gleichen aromatischen Kern befindet wie die mindestens eine Hydroxylgruppe. Bevorzugte Verbindungen sind (Mono)alkylphenole, wobei der Alkylrest in o-, m- oder p-Position zur Hydroxylgruppe stehen kann. Insbesondere bevorzugt sind trans-Alkylphenole, auch als 4-Alkylphenole bezeichnet, wobei der Alkylrest vorzugsweise 1 bis 10 Kohlenstoffatome aufweist und insbesondere ein tert.-Butylrest ist. Bevorzugt ist 4-tert.-Butylphenol. Erfindungsgemäß verwendbare mehrkernige aromatische Verbindungen sind beispielsweise β-Naphthol und α-Naphthol.

Die aromatischen Verbindungen, in denen mindestens eine Hydroxylgruppe und vorzugsweise ferner mindestens ein gegebenenfalls substituierter C₁₋₁₀-Alkylrest und/oder Alkoxyrest an einen aromatischen Kern gebunden ist, können auch mehrere aromatische Kerne aufweisen, die über einen Alkylenrest, vorzugsweise eine Methylengruppe verknüpft sind. Die verknüpfende Alkylengruppe, vorzugsweise Methylengruppe, kann einen oder mehrere Alkylsubstituenten aufweisen, die C₁₋₂₀-Alkylreste sein können und vorzugsweise C₁₋₁₀-Alkylreste, besonders bevorzugt sind Methyl-, Ethyl-, Propyl-, Isopropyl-, Butyl- oder tert.-Butylreste, sind.

Dabei kann jeder der aromatischen Kerne mindestens eine Hydroxylgruppe gebunden enthalten. Beispiele solcher Verbindungen sind Bisphenole, die in 4-Position über einen Alkylenrest, vorzugsweise einen Methylenrest, verknüpft sind.

Insbesondere bevorzugt umgesetzt wird im Rahmen des erfindungsgemäßen Verfahrens ein mit einem C₁₋₁₀-Alkylrest, vorzugsweise C₁₋₆-Alkylrest, substituiertes Phenol, wobei der Alkylrest gegebenenfalls mit einem aromatischen Rest substituiert ist, oder Gemische zweier oder mehrerer dieser Verbindungen.

In einer weiteren bevorzugten Ausführungsform dieses Verfahrens wird p-tert.-Butylphenol, Bis(p-hydroxyphenyl)dimethylmethan oder ein Gemisch davon umgesetzt.

### Aromatische Verbindungen, in denen mindestens eine Aminogruppe an einen aromatischen Kern gebunden ist

Mit dem erfindungsgemäßen Verfahren können ferner aromatische Verbindungen, in denen mindestens eine Aminogruppe an einen aromatischen Kern gebunden ist, zu den entsprechenden cycloaliphatischen Verbindungen hydriert werden, wobei auch Gemische zweier oder mehr dieser Verbindungen eingesetzt werden können. Dabei können die aromatischen Verbindungen einkernige oder mehrkernige aromatische Verbindungen sein. Die aromatischen Verbindungen enthalten mindestens eine Aminogruppe, die an einen aromatischen Kern gebunden ist. Vorzugsweise sind die aromatischen Verbindungen aromatische Amine oder Diamine. Die aromatischen Verbindungen können an dem aromatischen Kern oder den aromatischen Kernen oder an der Aminogruppe substituiert sein durch einen oder mehrere Alkyl- und/oder Alkoxyreste, vorzugsweise C₁₋₂₀-Alkylreste, insbesondere Methyl-, Ethyl-, Propyl-, Isopropyl-, Butyl-, Isobutyl-, tert.-Butylreste; unter den Alkoxyresten sind die C₁₋₈-Alkoxyreste, wie z.B. Methoxy-, Ethoxy-, Propoxy-, Isopropoxy-, Butoxy-, Isobutoxy-, tert.-Butoxy-Reste, bevorzugt. Der aromatische Kern oder die aromatischen Kerne sowie die Alkyl- und Alkoxyreste können gegebenenfalls durch Halogenatome, insbesondere Fluoratome, substituiert sein oder andere geeignete inerte Substituenten aufweisen.

Die aromatische Verbindung, in der mindestens eine Aminogruppe an einen aromatischen Kern gebunden ist, kann auch mehrere aromatische Kerne aufweisen, die über eine Alkylengruppe, vorzugsweise eine Methylengruppe, verknüpft sind. Die verknüpfende Alkylengruppe, vorzugsweise Methylengruppe, kann einen oder mehrere Alkylsubstituenten aufweisen, die C₁₋₂₀-Alkylreste sein können und vorzugsweise C₁₋₁₀-Alkylreste, besonders bevorzugt Methyl-, Ethyl-, Propyl-, Isopropyl-, Butyl-, sec.-Butyl- oder tert.-Butylreste, sind.

Die an den aromatischen Kern gebundene Aminogruppe kann ebenfalls durch einen oder zwei der vorstehend beschriebenen Alkylreste substituiert sein.

Besonders bevorzugte Verbindungen sind Anilin, Naphthylamin, Diaminobenzole, Diaminotoluole und Bis-p-aminophenylmethan oder Gemische davon.

### C = O-Gruppen enthaltende Verbindungen

Mit dem erfindungsgemäßen Verfahren können C = O-Gruppen enthaltende Verbindungen, also insbesondere Aldehyde, Ketone, Carbonsäuren und deren Derivate, wie z.B. Carbonsäureester, Carbonsäurehalogenide und Carbonsäureanhydride sowie Gemische aus zwei oder mehr der oben genannten Verbindungen umgesetzt, insbesondere hydriert werden.

Dabei werden insbesondere Aldehyde und Ketone, vorzugsweise solche mit 1 bis 20 C-Atomen, wie z.B. Formaldehyd, Acetaldehyd, Propionaldehyd, n-Butyraldehyd, Valeraldehyd, Caproaldehyd, Heptaldehyd, Phenylacetaldehyd, Acrolein, Crotonaldehyd, Benzaldehyd, o-Tolualdehyd, m-Tolualdehyd, p-Toluoaldehyd, Salicylaldehyd, Anisaldehyd, Vanillin, Zimtaldehyd, Aceton, Methylethylketon, 2-Pentanon, 3-Pentanon, 2-Hexanon, 3-Hexanon, Cyclohexanon, Isophoron, Methylisobutylketon, Mesityloxid, Acetophenon, Propiophenon, Benzophenon, Benzalaceton, Dibenzalaceton, Benzalacetophenon, Glykolaldehyd, Glyceraldehyd, Glyoxal, 2,3-Butandion, 2,4-Pentandion, 2,5-Hexandion, Terephthaldialdehyd, Glutardialdehyd, Diethylketon, Methylvinylketon, Acetylaceton, 2-Ethylhexanal oder Gemische aus zwei oder mehr davon eingesetzt.

Ferner kommen auch Polyketone, wie z.B. Ethylen/CO-Copolymere zum Einsatz.

Ferner lassen sich Carbonsäuren und Derivate davon umsetzen, wobei solche mit 1 bis 20 C-Atomen bevorzugt sind. Im einzelnen zu nennen sind:
Carbonsäuren, wie z.B. Ameisensäure, Essigsäure, Propionsäure, Buttersäure, Isobuttersäure, n-Valeriansäure, Trimethylessigsäure ("Pivalinsäure"), Capronsäure, Önanthsäure, Caprylsäure, Caprinsäure, Laurinsäure, Myristinsäure, Palmitinsäure, Stearinsäure, Acrylsäure, Methacrylsäure, Ölsäure, Elaidinsäure, Linolsäure, Linolensäure, Cyclohexancarbonsäure, Benzoesäure, Phenylessigsäure, o-Toluylsäure, m-Toluylsäure, p-Toluylsäure, o-Chlorbenzoesäure, p-Chlorbenzoesäure, o-Nitrobenzoesäure, p-Nitrobenzoesäure, Salicylsäure, p-Hydroxybenzoesäure, Anthranilsäure, p-Aminobenzoesäure, Oxalsäure, Malonsäure, Bernsteinsäure, Glutarsäure, Adipinsäure, Pimelinsäure, Korksäure, Azelainsäure, Sebacinsäure, Maleinsäure, Fumarsäure, Phthalsäure, Isophthalsäure, Terephthalsäure;
Carbonsäurehalogenide, wie z.B. die Chloride oder Bromide der oben genannten Carbonsäuren, insbesondere Acetylchlorid oder -bromid, Stearylchlorid oder -bromid und Benzoylchlorid oder -bromid, die insbesondere dehalogeniert werden;
Carbonsäureester, wie z.B. die C₁-C₁₀-Alkylester der oben genannten Carbonsäuren, insbesondere Methylformiat, Essigester, Buttersäurebutylester, Terephthalsäuredimethylester, Adipinsäuredimethylester, (Meth)acrylsäuremethylester, Butyrolacton, Caprolacton und Polycarbonsäureester, wie z.B. Polyacryl- und Polymethacrylsäureester und deren Copolymere und Polyester, wie z.B. Polymethylmethacrylat, wobei hier insbesondere Hydrogenolysen, also die Umsetzung von Estern zu den entsprechenden Säuren und Alkoholen, durchgeführt werden;
Carbonsäureanhydride, wie z.B. die Anhydride der oben genannten Carbonsäuren, insbesondere Essigsäureanhydrid, Propionsäureanhydrid, Benzoesäureanhydrid, Maleinsäureanhydrid.
Carbonsäureamide, wie z.B. Amide der oben genannten Carbonsäuren, wie z.B. Formamid, Acetamid, Propionamid, Stearamid, Terephthalsäureamid.

Ferner können auch Hydroxycarbonsäuren, wie z.B. Milch-, Äpfel-, Wein- oder Zitronensäure, oder Aminosäuren, wie z.B. Glycin, Alanin, Prolin und Arginin, umgesetzt werden.

### Nitrile

Ferner können auch Nitrile umgesetzt werden, vorzugsweise aliphatische und aromatische Mono- und Dinitrile, wie z.B. Acetonitril, Propionitril, Butyronitril, Stearinsäurenitril, Isocrotonsäurenitril, 3-Butennitril, Propinnitril, 3-Butinnitril, 2, 3-Butadiennitril, 2,4-Pentadiennitril, 3-Hexen-1,6 dinitril, Chloracetonitril, Trichloracetonitril, Milchsäurenitril, Phenol-Acetonitril, 2-Chlorbenzonitril, 2,6-Dichlorbenzonitril, Isophthalsäuredinitril und Terephthalsäuredinitril, insbesondere von aliphatischen α,ω-Dinitrilen, wie z.B. Bernsteinsäuredinitril, Glutarsäuredinitril, Adipinsäuredinitril, Pimelinsäuredinitril und Korksäuredinitril, oder Aminonitrilen, wie z.B. 4-Aminobutansäurenitril, 5-Aminopentansäurenitril, 6-Aminohexansäurenitril, 7-Aminoheptansäurenitril und 8-Aminooctansäurenitril.

Ferner lassen sich mit dem erfindungsgemäßen Verfahren folgende Umsetzungen durchführen:
die Hydrierung von aromatischen Verbindungen, wie z.B. Benzol, Toluolen, Xylolen, Naphthalinen sowie substituierten Derivaten davon, zu den entsprechenden alicyclischen Verbindungen; die Hydrierung von Alkenen oder Alkinen, wie z.B. Ethylen, Propylen, 1-, 2-Buten, 1-, 2-, 3- und 4-Octen, Butadien und Hexatrien zu den entsprechenden Alkanen; die Hydrierung von Nitroalkanen, wie z.B. Nitroethan, Nitromethan, Nitropropan und 1,1-Dinitroethan zu den entsprechenden Aminen;
die Hydrierung von Iminen, wie z.B. Chinoniminen, Ketiminen, Keteniminen oder aliphatischen Iminen, wie z.B. Propanimin, Hexanimin; die Dehalogenierung von Halogenatome enthaltenden organischen Verbindungen, insbesondere von aromatischen halogenhaltigen Verbindungen, wie z.B. Chlor- und Brombenzol, Brom- und Chlortoluolen sowie Chlor- und Bromxylolen, wobei dort auch jeweils mit mehreren Halogenatomen substituierte Verbindungen eingesetzt werden können; die aminierende Hydrierung von z.B. Alkoholen, wie z.B. Vinylalkohol.

Ferner lassen sich Oxime mit dem erfindungsgemäßen Verfahren umsetzen oder sekundäre Amine aus Ketonen und primären Aminen herstellen.

Die erfindungsgemäßen Katalysatoren können auch für Hydrierungen, Dehydrierungen, Hydrogenolysen, aminierende Hydrierungen und Dehalogenierungen von großen Molekülen, vorzugsweise von Polymeren, verwendet werden.

Demgemäß betrifft die vorliegende Erfindung auch ein Verfahren zur Umsetzung eines Polymers, das mindestens eine katalytisch umsetzbare Gruppe aufweist, in Gegenwart des oben beschriebenen Katalysators, wobei die Hydrierung von C=O-Gruppen aufweisenden Polymeren, wie z.B. Polyester von Dicarbonsäuren, ungesättigten Monocarbonsäuren, wie z.B. Poly-(meth)acrylaten, Olefin/CO-Copolymeren oder Polyketonen bevorzugt ist.

Insbesondere betrifft die vorliegende Erfindung ein Verfahren zur Hydrierung eines Polymers, das mindestens eine C=O-Gruppe aufweist, in Gegenwart des oben genannten Katalysators.

Der Begriff *"Polymer, das mindestens eine katalytisch umsetzbare Einheit aufweist*" steht für alle Polymere, die derartige Einheiten aufweisen, insbesondere für Polymere, die Einheiten der folgenden Strukturen (I) bis (VIII), wie oben bezüglich der monomeren Verbindungen definiert, oder ein Halogenatom aufweisen. Dabei ist es selbstverständlich, daß die hier in Rede stehenden Polymere die jeweilige Einheit mindestens einmal enthalten und sich auch innerhalb des erfindungsgemäß umgesetzten Polymers eine oder mehrere Einheiten von zwei oder mehr der Strukturen (I) bis (VIII) befinden können.

Das mittlere Molekulargewicht der im erfindungsgemäßen Verfahren umzusetzenden Polymere beträgt im allgemeinen ungefähr 500 bis ungefähr 500.000, vorzugsweise ungefähr 1.000 bis ungefähr 100.000 und weiter bevorzugt ungefähr 1.000 bis ungefähr 50.000.

Als Beispiele für im Rahmen des erfindungsgemäßen Verfahrens umsetzbare, vorzugsweise hydrierbare Polymere sind die folgenden zu nennen:
Polymere mit C―C-Doppelbindungen, wie z.B. Polybutadiene, wie z.B. Poly(2,3-dimethylbutadien), Polyisopren, Polyacetylene und Polycyclopenta- und -hexadiene; Polymere mit C―C-Dreifachbindungen, wie z.B. Polydiacetylene; Polymere mit aromatischen Gruppen, wie z.B. Polystyrol, Acrylnitril-Butadien-Styrol-Terpolymere und Styrol-Acrylnitril-Copolymere; Polymere mit C―N-Dreifachbindungen, wie z.B. Polyacrylnitril, Polyacrylnitril-Copolymere mit z.B. Vinylchlorid, Vinylidenchlorid, Vinylacetat oder (Meth)acrylsäureestern oder Gemischen aus zwei oder mehr davon als Comonomere; Polymere mit C―O-Doppelbindungen, wie z.B. Polyester, Polyacrylamide, Polyacrylsäuren, Polyharnstoffe und Polyketone; Polymere mit C―S-Doppelbindungen, wie z.B. Polysulfone und Polyethersulfone; halogenhaltige Polymere, wie z.B. Polyvinylchlorid und Polyvinylidenchlorid; sowie Nitrogruppen enthaltende Polymere, die durch Nitrierung von z.B. Polyolefinen durch polymeranaloge Umsetzung erhalten werden können.

Beispiele für im Rahmen der vorliegenden Erfindung bevorzugt eingesetzte Polymere schließen Polyisopren, Polybutadien, Ethylen/CO-Copolymere, Propylen/CO-Copolymere, Polymethyl(meth)acrylat, Polyterephthalate, Polyadipate usw. ein.

Im allgemeinen findet eine vollständige Umsetzung der eingesetzten Edukte statt. Die Umsetzung, vorzugsweise Hydrierung kann jedoch auch so durchgeführt werden, daß durch geeignete Wahl von Temperatur, z.B. H₂-Druck und H₂-Menge lediglich eine Art von z.B. hydrierbaren Gruppen hydriert wird, während die andere Art von hydrierbaren Gruppen nicht hydriert wird.

Besonders geeignet ist das erfindungsgemäße Verfahren zur Umsetzung, vorzugsweise Hydrierung von Polymeren, die Einheiten verschiedener Strukturen, wie oben definiert, enthalten, z.B. Polymere, die sowohl C-C-Mehrfachbindungen als auch C=O-Gruppen aufweisen, da die im Rahmen des erfindungsgemäßen Verfahrens verwendeten Katalysatoren in der Lage sind, zunächst die C-C-Mehrfachbindungen selektiv umzusetzen, d.h. eine Umsetzung dieser Gruppen von ungefähr 90 bis 100% zu erreichen, während zunächst gleichzeitig die C=O-Gruppen zu weniger als 25% und im allgemeinen in einem Bereich von 0 bis ungefähr 7% umgesetzt, z.B. hydriert werden.

Nach Beendigung der Umsetzung der in den Polymeren vorhandenen C-C-Mehrfachbindungen ist es selbstverständlich möglich, durch weitere Zufuhr von Wasserstoff auch die restlichen, im Polymer vorhandenen ungesättigten Gruppen, wie z.B. C=O-Gruppen, nahezu quantitativ umzusetzen, z.B. zu hydrieren.

Das erfindungsgemäße Verfahren kann sowohl für bereits isolierte als auch für lebende Polymere eingesetzt werden.

### KATALYSATOREN

Das erfindungsgemäße Verfahren wird in Gegenwart eines Katalysators durchgeführt, der eine in situ auf einem Träger abgeschiedene, homogene Rutheniumverbindung umfaßt. Die Herstellung des Katalysators kann dadurch erfolgen, daß eine homogene Rutheniumverbindung zusammen mit der organischen Verbindung in einen Reaktor eingebracht wird und sich während der Umsetzung auf einen im Reaktor befindlichen Träger abscheidet.

Ferner kann die homogene Rutheniumverbindung auch vor der Umsetzung in den Reaktor eingebracht werden und sich während derselben auf einen im Reaktor befindlichen Träger abscheiden.

Der im Rahmen der vorliegenden Anmeldung verwendete Begriff "in situ" bedeutet, daß der Katalysator nicht separat hergestellt und getrocknet wird und dann als sozusagen fertiger Katalysator in den Reaktor eingebracht wird, sondern daß der Katalysator im Rahmen der vorliegenden Erfindung im Reaktor während der eigentlichen Hydrierung gebildet wird.

Der im Rahmen der vorliegenden Anmeldung verwendete Begriff "homogene Rutheniumverbindung" bedeutet, daß die verwendete Rutheniumverbindung in dem sie umgebenden Medium, d.h. der eingesetzten, noch umzusetzenden organischen Verbindung oder in einem Gemisch dieser Verbindungen mit mindestens einem Lösungsmittel löslich ist.

Als Rutheniumverbindungen kommen dabei vor allem Nitrosylnitrate und Nitrate, aber auch Halogenide, Carbonate, Carboxylate, Acetylacetonate, Chlor-, Nitrido- und Aminkomplexe sowie Oxidhydrate oder deren Gemische zum Einsatz. Bevorzugte Verbindungen sind Rutheniumnitrosylnitrat, Ruthenium(III)chlorid, Ruthenium(III)nitrat und Rutheniumoxidhydrat.

Obwohl die Menge der im Rahmen des erfindungsgemäßen Verfahrens auf den oder die Träger aufgebrachten Rutheniumverbindung nicht in besonderer Weise beschränkt ist, wird unter den Gesichtspunkten einer ausreichenden katalytischen Aktivität und der Wirtschaftlichkeit des Verfahrens, das Rutheniumsalz oder der Rutheniumkomplex in einer solchen Mengen auf den oder die Träger aufgebracht, daß sich 0,01 bis 30 Gew.-%, bezogen auf das Gesamtgewicht des Katalysators an Ruthenium auf dem oder den Trägern abscheidet. Weiter bevorzugt beträgt diese Menge 0,2 bis 15 Gew.-%, insbesondere bevorzugt etwa 0,5 Gew.-%.

### TRÄGER

Die geometrische Form des Trägers bzw. Katalysators ist nicht besonders beschränkt und kann in weiten Bereichen variiert werden und z.B. als strukturierte Gewebepackung, als Monolith, als Ring, als Helix, usw. ausgeführt sein.

Die im Reaktor befindlichen Träger sind vorzugsweise Metallnetze und Metallringe sowie Steatitkörper, wie sie unter anderem in der EP-A-0 564 830 und der EP-A-0 198 435 beschrieben werden. Im folgenden sollen dennoch kurz die im Rahmen der vorliegenden Erfindung besonders bevorzugten Träger und deren Herstellung erläutert werden.

Besonders bevorzugt werden metallische Trägermaterialien, wie beispielsweise die Edelstähle mit Werkstoffnummern 1.4767, 1.4401, 2.4610, 1.4765, 1.4847, 1.4301 usw. verwendet, da sie vor der Beschichtung mit den Aktivkomponenten durch eine Temperung eine Aufrauhung ihrer Oberfläche erhalten können. Insbesondere bevorzugt werden als Netzmaterial Kanthal (Werkstoffnr. 1.4767) bzw. Metalle, die Aluminium enthalten, eingesetzt. Kanthal ist eine Legierung, die zu ungefähr 75 Gew.-% Fe, ungefähr 20 Gew.-% Cr und ungefähr 5 Gew.-% Al enthält. Für die Temperung werden die oben erwähnten metallischen Träger bei Temperaturen von 600 bis 1100, vorzugsweise 800 bis 1000 °C, eine bis zwanzig, vorzugsweise eine bis zehn, Stunden an der Luft erhitzt und wieder abgekühlt. Diese Vorbehandlung ist in der EP-A-0 564 830 beschrieben und für die Aktivität des Katalysators entscheidend, da ohne diese Temperungsbehandlung praktisch kein Ruthenium in situ auf den metallischen Träger abgeschieden werden kann. Nach dieser Trägerbehandlung bei erhöhter Temperatur wird der Träger entweder mit der Rutheniumverbindung beschichtet oder direkt in den Reaktor eingesetzt.

In einer weiter bevorzugten Ausführungsform können die oben beschriebenen Träger mit einer Schicht eines Palladiummetalls, wie z.B. Ni, Pd, Pt, Rh, vorzugsweise Pd, in einer Dicke von ungefähr 0,5 bis ungefähr 10 nm, insbesondere circa 5 nm, bedampft werden, wie dies ebenfalls in der bereits oben erwähnten EP-A-0 564 830 beschrieben wird. Ferner kann das oben genannte Metall der VIII. Nebengruppe (Palladiummetall) auch durch Tränken aufgebracht werden. Der Metallgehalt beträgt dabei im allgemeinen ungefähr 1 bis ungefähr 5% der Trägeroberfläche.

Die erfindungsgemäß verwendeten Katalysatoren können zur Beeinflussung ihrer katalytischen Eigenschaften noch mit einem oder mehreren anderen Metallen insbesondere der I., VI., VII. oder VIII. Nebengruppe des Periodensystems promotiert werden, wobei Chrom, Eisen, Cobalt, Tantal, Molybdän, Iridium, Renium sowie die oben genannten Platinmetalle zu nennen sind. Typischerweise beträgt der Gehalt der Promotoren auf dem Katalysator ungefähr 0,01 bis ungefähr 15 Gew.-%, vorzugsweise ungefähr 0,1 bis ungefähr 5 Gew.-%, bezogen auf das Gesamtgewicht des Katalysators.

Wie sich auch aus den erfindungsgemäßen Beispielen ergibt, wird im Rahmen der vorliegenden Erfindung als Katalysator insbesondere ein Netz aus getempertem Kanthal, auf das eine Pd-Schicht mit einer Dicke von ungefähr 5 nm aufgedampft wurde, als Träger verwendet.

Es können jedoch auch übliche Katalysatorträgersysteme, wie zum Beispiel Aktivkohle, Siliciumcarbid, Aluminiumoxid, Siliciumdioxid, Titandioxid, Zirconiumdioxid, Magnesiumoxid, Zinkoxid oder deren Gemische, jeweils in Kugel-, Strang- oder Ringform, eingesetzt werden. Unter diesen besonders bevorzugt sind Aluminiumoxid und Zirconiumdioxid. Dabei ist die Porengröße sowie die Porenverteilung völlig unkritisch. Es können bimodale und auch alle anderen Arten von Trägern eingesetzt werden. Vorzugsweise sind die Träger makroporös.

Die erfindungsgemäß verwendeten Katalysatoren zeigen eine hohe Reaktivität (hohe Turn-Over-Zahl), Selektivität und Standzeit. Unter Einsatz der erfindungsgemäß verwendeten Katalysatoren werden in der Umsetzung die Umsetzungsprodukte in hoher Ausbeute und Reinheit gewonnen, wobei eine nachfolgende Reinigung überflüssig ist. Der Umsatz ist praktisch quantitativ, z.B. liegt der Restaromatenteil vorzugsweise unter 0,01 Gew.-%, bezogen auf die gesamte Produktmenge. Das erhaltene Produkt kann in einer bevorzugten Ausführungsform der vorliegenden Erfindung somit direkt einer Weiterverarbeitung zugeführt werden, ohne gereinigt werden zu müssen.

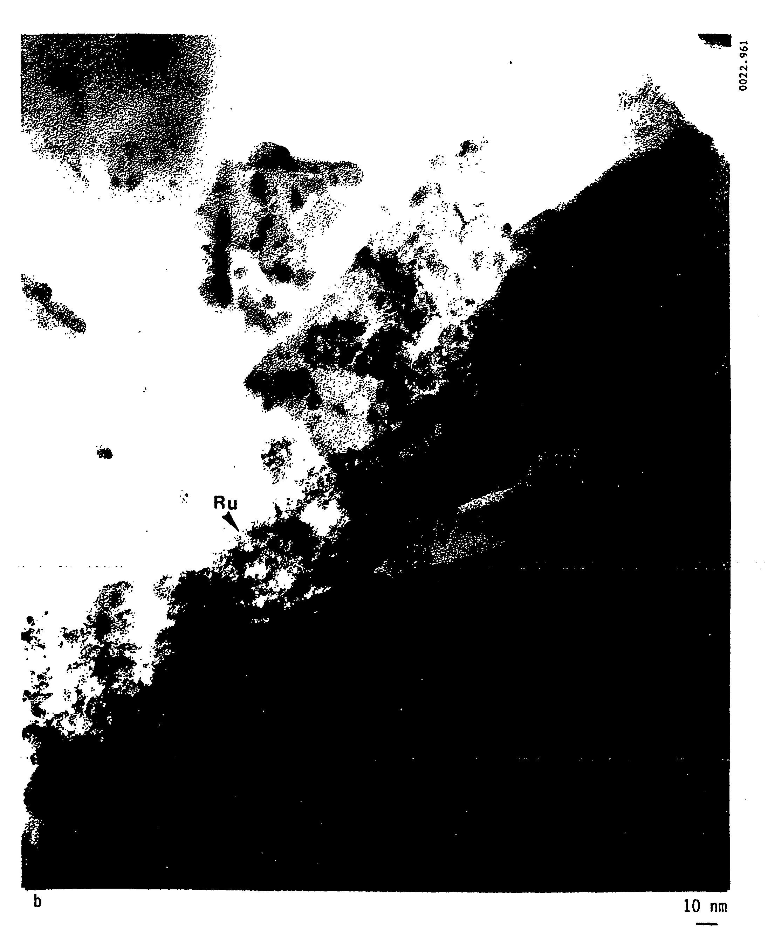

Fig. 1 zeigt eine Transmissions-Elektronenmikroskopie-Aufnahme eines erfindungsgemäßverwendeten Katalysators. Wie daraus ersichtlich, besitzt der erfindungsgemäßverwendete Katalysator bezüglich des aufgebrachten Rutheniums eine hohe Porosität, die höher ist als beim Aufbringen von Ruthenium auf einen derartigen Träger durch herkömmliche Verfahren, wie z.B. Tränken. Ohne an eine bestimmte Theorie gebunden zu sein, erscheint diese besondere Struktur für die vorteilhaften Eigenschaften des erfindungsgemäßverwendeten Katalysators verantwortlich zu sein.

Die Unterschiede der Struktur eines Metalls, das durch Tränken auf den Träger aufgebracht wurde, und eines Metalls, das wie oben für den erfindungsgemäßverwendeten Katalysator ausgeführt aufgebracht wurde, ergibt sich unmittelbar aus einem Vergleich der Struktur der Pd-Schicht, die durch Tränken aufgebracht wurde, mit der Ru-Schicht.

### LÖSUNGS- ODER VERDÜNNUNGSMITTEL

Beim erfindungsgemäßen Verfahren kann die katalytische Umsetzung unter Abwesenheit eines Lösungs- oder Verdünnungsmittels durchgeführt werden, d.h. es ist nicht erforderlich, die Umsetzung in Lösung durchzuführen.

Vorzugsweise wird jedoch ein Lösungs- oder Verdünnungsmittel eingesetzt. Als Lösungs- oder Verdünnungsmittel kann jedes geeignete Lösungs- oder Verdünnungsmittel eingesetzt werden. Die Auswahl ist dabei nicht kritisch. Beispielsweise können die Lösungs- oder Verdünnungsmittel auch geringe Mengen an Wasser enthalten.

Bei der Umsetzung einer aromatischen Verbindung, in der mindestens eine Hydroxylgruppe an einen aromatischen Kern gebunden ist, schließen Beispiele geeigneter Lösungs- oder Verdünnungsmittel die folgenden ein:

Geradkettige oder cyclische Ether, wie beispielsweise Tetrahydrofuran oder Dioxan, sowie aliphatische Alkohole, in denen der Alkylrest vorzugsweise 1 bis 10 Kohlenstoffatome, insbesondere 3 bis 6 Kohlenstoffatome, aufweist.

Beispiele bevorzugt verwendbarer Alkohole sind i-Propanol, n-Butanol, i-Butanol und n-Hexanol.

Gemische dieser oder anderer Lösungs- oder Verdünnungsmittel können ebenfalls verwendet werden.

Bei der Umsetzung einer aromatischen Verbindung, in der mindestens eine Aminogruppe an einen aromatischen Kern gebunden ist, schließen Beispiele geeigneter Lösungs- oder Verdünnungsmittel die folgenden ein:

Geradkettige oder cyclische Ether, wie beispielsweise Tetrahydrofuran oder Dioxan, sowie Ammoniak und Mono- oder Dialkylamine, in denen der Alkylrest vorzugsweise 1 bis 3 Kohlenstoffatome aufweist.

Gemische dieser oder anderer Lösungs- oder Verdünnungsmittel können ebenfalls verwendet werden.

In beiden obigen Ausführungsformen ist die Menge des eingesetzten Lösungs- oder Verdünnungsmittels nicht in besonderer Weise beschränkt und kann je nach Bedarf frei gewählt werden, wobei jedoch solche Mengen bevorzugt sind, die zu einer 10 bis 70 Gew.-%igen Lösung der zur Hydrierung vorgesehenen Verbindung führen.

Besonders bevorzugt wird im Rahmen des erfindungsgemäßen Verfahrens das bei der Umsetzung dieses Verfahrens gebildete Produkt als Lösungsmittel eingesetzt, gegebenenfalls neben anderen Lösungs- oder Verdünnungsmitteln. In diesem Fall kann ein Teil des bei der Umsetzung gebildeten Produktes den umzusetzenden Verbindungen beigemischt werden. Bezogen auf das Gewicht der zur Umsetzung vorgesehenen organischen Verbindungen wird vorzugsweise die 1 bis 30-fache, besonders bevorzugt die 5 bis 20-fache, insbesondere die 5 bis 10-fache Menge des Umsetzungsproduktes als Lösungs- oder Verdünnungsmittel zugemischt.

Auch für die anderen erfindungsgemäß umsetzbaren Verbindungen gilt das oben Gesagte, wobei auch dort keine Beschränkung bezüglich der Lösungs- und Verdünnungsmittel bestehen.

### UMSETZUNG

Nachfolgend wird die Umsetzung am Beispiel einer Hydrierung beschrieben, wobei - sofern eine Dehydrierung oder eine Oxidation durchgeführt werden soll - anstelle von Wasserstoff bzw. wasserstoffhaltigen Gasen gasförmige Kohlenwasserstoffe bzw. sauerstoffhaltige Gase unter den nachstehend beschriebenen Bedingungen verwendet werden können.

Die Hydrierung wird bei geeigneten Drücken und Temperaturen durchgeführt. Bevorzugt sind Drücke oberhalb von 50 bar, vorzugsweise von 100 bis 300 bar. Bevorzugte Temperaturen liegen in einem Bereich von 50 bis 250°C, vorzugsweise zwischen 150 und 220°C.

Das Hydrierungsverfahren kann kontinuierlich oder in Art eines Batch-Verfahrens durchgeführt werden. Beim kontinuierlichen Verfahren kann dabei ein Teil des den Reaktor verlassenden Hydrierungsproduktes dem Reaktorzulauf vor dem Reaktor zugeführt werden. Dabei wird eine solche Menge des den Reaktor verlassenden Hydrierungsprodukt als Lösungsmittel rückgeführt, daß die im Unterabschnitt "Lösungs- und Verdünnungsmittel" genannten Mengenverhältnisse erreicht werden. Die verbleibende Menge an Hydrierungsprodukt wird abgezogen.

Bei kontinuierlicher Prozeßführung beträgt die Menge der zur Hydrierung vorgesehenen Verbindung bzw. Verbindungen vorzugsweise ungefähr 0,05 bis ungefähr 3 l pro Liter Katalysator pro Stunde, weiter bevorzugt ungefähr 0,1 bis ungefähr 1 l pro Liter Katalysator pro Stunde.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet.

Je nach Reaktionsbedingungen kann bei den zusätzlich durch mindestens einen ggf. substituierten C₁₋₁₀- und/oder -Alkoxyrest substituierten Phenolen und Aminen je nach Reaktionsbedingungen (Temperatur, Lösungsmittel) das erhaltene Isomerenverhältnis von cis- zu trans-konfigurierten Produkten in einem weiten Bereich variiert werden.

Sofern eine aromatische Verbindung, in der mindestens eine Aminogruppe an einen aromatischen Kern gebunden ist, mittels des erfindungsgemäßen Katalysators hydriert werden soll, wird die Hydrierung vorzugsweise in Gegenwart von Ammoniak oder Dialkylaminen, beispielsweise Methylamin, Ethylamin, Propylamin oder Dimethylamin, Diethylamin oder Dipropylamin durchgeführt. Dabei werden geeignete Mengen an Ammoniak oder Mono- oder Dialkylamin eingesetzt, die vorzugsweise bei ungefähr 0,5 bis ungefähr 50 Gewichtsteilen, besonders bevorzugt bei ungefähr 1 bis ungefähr 20 Gewichtsteilen, jeweils bezogen auf 100 Gewichtsteile der zur Hydrierung vorgesehenen Verbindung oder Verbindungen, eingesetzt. Besonders bevorzugt werden wasserfreier Ammoniak oder wasserfreie Amine eingesetzt.

Für Oxidationen wird im allgemeinen Luft oder reiner Sauerstoff verwendet. Für Dehydrierungen werden die üblicherweise verwendeten Kohlenwasserstoffe, insbesondere Methan bzw. Erdgas, eingesetzt.

Die Erfindung wird im folgenden anhand von einigen Ausführungsbeispielen näher erläutert, wobei sich Beispiele 1 bis 6 auf die Hydrierung einer aromatischen Verbindung, in der mindestens eine Hydroxylgruppe an einen aromatischen Kern gebunden ist, beziehen, sich Beispiele 7 bis 15 auf die Hydrierung einer aromatischen Verbindung, in der mindestens eine Aminogruppe an einen aromatischen Kern gebunden ist, beziehen, und Beispiele 16 bis 20 sich auf die Hydrierung von Aldehyden, Ketonen und Estern beziehen.

### Beispiel 1

In einem 3,5 l-Autoklaven wurde ein Netz aus getempertem Kanthal, auf das eine Pd-Schicht mit einer Dicke von 5 nm aufgedampft worden war, mit 1 mm Maschendurchmesser angebracht. In der ersten Fahrt wurden 2000 ml p-tert.-Butylphenol mit 200 mg Rutheniumnitrosylnitrat in den Autoklaven gegeben. Anschließend wurde der Ansatz 60 Minuten bei 200 bar Wasserstoffdruck und 180°C hydriert. Der Reaktionsaustrag war farblos und frei von Rutheniumspuren. Er bestand zu 98,5 % aus p-tert.-Butylcyclohexanol. In einer zweiten Fahrt wurden analog 2000 ml p-tert.-Butylphenol ohne Rutheniumzusatz mit Wasserstoff umgesetzt. Der farblose Reaktionsaustrag war frei von Rutheniumspuren und bestand zu 99 % aus p-tert.-Butylcyclohexanol.

### Beispiel 2

Die Hydrierung wurde analog Beispiel 1 durchgeführt. Als Rutheniumverbindung wurde Rutheniumoxihydrat verwendet. Der klare Reaktionsaustrag war frei von p-tert.-Butylphenol und Rutheniumspuren. Er enthielt 96 % p-tert.-Butylcyclohexanol. In dem Autoklaven wurden anschließend nochmals 30 Fahrten ohne weiteren Rutheniumzusatz durchgeführt. Umsatz und Selektivität blieben während allen Fahrten konstant.

### Beispiel 3

Die Hydrierung wurde analog Beispiel 1 durchgeführt. Als Phenolverbindung wurde 50 g Bisphenol A, gelöst in 100 ml Tetrahydrofuran, und 50 mg Rutheniumnitrosylnitrat eingesetzt. Die Umsetzung wurde bei 200°C und 200 bar Wasserstoffdruck 90 Minuten lang durchgeführt. Die eingesetzte Phenolverbindung wurde innerhalb dieser Zeit mit einer Selektivität von 98 % vollständig umgesetzt.

### Beispiel 4

In einen 3,5 l-Autoklaven mit Korbeinsatz aus V₂A-Stahl wurden 120 g Steatitkugeln, die zuvor in eine 15 %-ige salpetersaure Rutheniumnitrosylnitratlösung getaucht worden waren, eingefüllt. Danach wurden 2000 ml p-tert.-Butylphenol in den Autoklaven gegeben und der Ansatz 60 Minuten bei 200 bar Wasserstoffdruck und 180°C hydriert. Der Reaktionsaustrag war farblos und frei von Rutheniumspuren. Er bestand zu 97 % aus p-tert.-Butylcyclohexanol.

### Beispiel 5

Ein 3 l-Rohrreaktor wurde mit Metallringen (Durchmesser 3 mm; hergestellt und vertrieben durch die Firma Raschig) gefüllt. Bei 200 bar Wasserstoffdruck und 180°C wurden 3000 ml p-tert.-Butylphenol, in denen 500 mg Rutheniumnitrosylnitrat gelöst worden waren, innerhalb von 60 Minuten in den Reaktor eingebracht. Danach wurde pro Stunde 1500 ml frisches p-tert.-Butylphenol ohne weiteren Rutheniumzusatz zugefahren. Der Umsatz im Rohaustrag war quantitativ, und der Gehalt an p-tert.-Butylcyclohexanol betrug 98 %.

### Beispiel 6

Unterschiedliche Rutheniumverbindungen wurden analog Beispiel 1 zur Hydrierung von p-tert.-Butylphenol eingesetzt. Das dabei verwendete Kanthalnetz wurde vorher nicht mit Pd bedampft. Die Ergebnisse sind in folgender Tabelle festgehalten:

| **Verbindung** | **Umsatz** | **Selektivität** |
|---|---|---|
| Ru(NO₃)₂ | 95 % | 97 % |
| Ru(acac) | 92 % | 96 % |
| RuCl₃ | 85 % | 97 % |

### Beispiel 7

In einem 3,5 l-Autoklaven wurde ein Netz aus getempertem Kanthal, auf das eine Pd-Schicht mit einer Dicke von 5 nm aufgedampft worden war, mit 1 mm Maschendurchmesser angebracht. 200 mg Rutheniumnitrosylnitrat wurden in 2000 ml Anilin gelöst und in den Autoklaven gegeben. Anschließend wurde der Ansatz 60 Minuten lang bei 200 bar Wasserstoffdruck und 180 °C hydriert. Der Reaktionsaustrag war farblos und frei von Rutheniumspuren. Er bestand zu 99,9 % aus Cyclohexylamin. Anschließend wurden 2000 ml Anilin ohne Zusatz von Rutheniumverbindungen im gleichen Autoklaven mit Wasserstoff umgesetzt. Der farblose Reaktionsaustrag war frei von Rutheniumspuren und bestand zu 99,8 % aus Cyclohexylamin.

### Beispiel 8

Die Hydrierung wurde analog Beispiel 7 durchgeführt. Als Rutheniumverbindung wurde Rutheniumoxihydrat verwendet. Der klare Reaktionsaustrag war frei von Anilin und Rutheniumspuren. Er enthielt 96 % Cyclohexylamin und 4 % Dicyclohexylamin. In dem Autoklaven wurden anschließend noch 30 Fahrten ohne weiteren Rutheniumzusatz durchgeführt. Umsatz und Selektivität blieben während allen Fahrten konstant.

### Beispiel 9

Die Hydrierung wurde analog Beispiel 7 durchgeführt. Als Aminverbindung wurden 50 g Toluylendiamin, gelöst in 100 ml Tetrahydrofuran, und 50 mg Rutheniumhydrosylnitrat eingesetzt. Es wurde 90 Minuten lang bei 200°C und 200 bar Wasserstoffdruck umgesetzt. Der Umsatz war quantitativ. Die Selektivität betrug 98 %.

### Beispiel 10

Die Hydrierung wurde analog Beispiel 7 durchgeführt. Als Aminverbindung wurde Toluidinbase eingesetzt. Der Umsatz war vollständig, die Selektivität betrug 98%.

### Beispiel 11

Die Hydrierung wurde analog Beispiel 7 durchgeführt. Als Aminverbindung wurde Phenylbase eingesetzt. Der Umsatz war vollständig, die Selektivität betrug 97%.

### Beispiel 12

In einem 3,5 l-Autoklaven mit Korbeinsatz aus V₂A-Stahl wurden 120 g Steatitkugeln, die zuvor in eine 15 %-ige salpetersaure Rutheniumnitrosylnitratlösung getaucht worden waren, eingefüllt. Danach wurden 2000 ml Anilin in den Autoklaven gegeben und der Ansatz 60 Minuten lang bei 200 bar Wasserstoffdruck und 180°C hydriert. Der Reaktionsaustrag war farblos und frei von Rutheniumspuren. Er bestand zu 99 % aus Cyclohexylamin.

### Beispiel 13

Ein 3 l-Rohrreaktor wurde mit Metallringen (Durchmesser 3 mm, hergestellt und vertrieben von der Firma Raschig) gefüllt. Bei 200 bar Wasserstoffdruck und 180°C wurden 3000 ml Anilin, in denen 500 mg Rutheniumnitrosylnitrat gelöst worden waren, innerhalb von 60 Minuten in den Reaktor eingebracht. Danach wurden pro Stunde 1500 ml frisches Anilin ohne Rutheniumzusatz zugefahren. Der Umsatz im Rohaustrag war quantitativ. Der Gehalt an Cyclohexylamin betrug 99,5 %.

### Beispiel 14

3 l Steatitkugeln wurden mit einer 15 %-igen salpetersauren Rutheniumnitrosylnitratlösung getränkt und anschließend bei 200°C mit Wasserstoff behandelt. Die so präparierten Kugeln wurden in einen 3 l Rohrreaktor gefüllt und wie in Beispiel 13 zur Hydrierung von Anilin ohne weiteren Rutheniumzusatz eingesetzt.

### Beispiel 15

Analog Beispiel 8 wurden unterschiedliche Rutheniumverbindungen zur Hydrierung von Anilin eingesetzt. Die Ergebnisse sind in folgender Tabelle gezeigt.

| **Verbindung** | **Umsatz** | **Selektivität** |
|---|---|---|
| Ru(NO₃)₂ | 95 % | 97 % |
| Ru(acac) | 92 % | 96 % |
| RuCl₃ | 85 % | 97 % |

Die Umsetzung der nun folgenden Verbindungen wurde mit folgenden Katalysatoren durchgeführt:

### Katalysator I

Ein Edelstahlgewebe mit der Werkstoff-Nr. 1.4767 aus Fe, Cr und Al wurde in einem Ultraschallbad von anhaftendem Öl und Fett gereinigt und 5 h lang bei 900 °C in einer Muffel an der Luft erhitzt. Das so erhaltene Gewebe wurde teilweise mittels einer Zahnradwalze verformt und danach das gewellte Gewebestück mit einem glatten Gewebestück zusammengerollt. Der so erhaltene zylindrische Monolith wurde paßgenau in einen kontinuierlichen Hydrierreaktor eingebaut.

### Katalysator II

Ein Edelstahlgewebe mit der Werkstoff-Nr. 1.4767 aus Fe, Cr und Al wurde in einem Ultraschallbad von anhaftendem Öl und Fett gereinigt und 5 h lang bei 900 °C in einem Muffelofen an der Luft erhitzt. Nach dem Erkalten wurde das Gewebe in einer kontinuierlich arbeitenden Elektronenstrahlbedampfungsanlage beidseitig mit einer 4 nm dicken Palladiumschicht bedampft (die Schichtdicke wurde mit einem Schwingquarz gemessen). Das so erhaltene Gewebe wurde teilweise mittels einer Zahnradwalze verformt und danach das gewellte Gewebestück mit einem glatten Gewebestück zusammengerollt. Der so erhaltene zylindrische Monolith wurde paßgenau in einen kontinuierlichen Hydrierreaktor eingebaut.

### Beispiel 16

In einem Rohrreaktor (l = 2500 mm, Ø = 45 mm) wurde Katalysator I in Form von Metallnetzrollen eingebaut, der Reaktor mit n-Butanol gefüllt und bei 3 · 10⁶ Pa (30 bar) Wasserstoffdruck auf 180 °C aufgeheizt. Anschließend wurde bei einer Umlaufmenge von 50 l/h kontinuierlich eine Menge von 1 kg/h n-Butyraldehyd in den Reaktor gefahren. In den ersten 5 l Butyraldehyd befanden sich 13 g Rutheniumnitrosylnitrat. Der erhaltene Reaktionsaustrag war farblos und frei von Ruthenium.

Die gaschromatographische Auswertung ergab einen Umsatz von 99,4% und eine Selektivität bezüglich n-Butanol von 99,7%, jeweils bezogen auf die eingesetzte Menge von n-Butyraldehyd.

### Beispiel 17

In einem 3,5 l-Autoklaven wurde ein Metallnetz mit Katalysator II (ungefähr 40.000 cm³) sowie 700 g eines Ethylen/CO-Copolymeren (Mw = 5.000, CO-Gehalt 35%), gelöst in 1.300 g THF und 500 mg Rutheniumnitrosilnitrat eingefüllt.

Anschließend wurde der Ansatz bei 180 °C und 2 · 10⁷ Pa (200 bar) Wasserstoffdruck 5 h lang hydriert. Der Umsatz zum gewünschten Polyalkohol lag bei 93%, bezogen auf die eingesetzte Menge des Copolymeren.

### Beispiel 18

In einem 3,5 l-Autoklaven wurde ein Metallnetz mit Katalysator II eingesetzt und 2.000 g Benzaldehyd und 500 mg Rutheniumnitrosylnitrat eingefüllt. Anschließend wurde der Ansatz bei 180 °C und 2 · 10⁷ Pa (200 bar) Wasserstoffdruck 10 h lang hydriert. Der Umsatz zum gewünschten Cyclohexylmethanol lag bei 100% bei einer Selektivität von 96,2%, jeweils bezogen auf die eingesetzte Menge an Benzaldehyd.

### Beispiel 19

In einem 3,5 l-Autoklaven wurde ein Metallnetz mit Katalysator II eingesetzt und 2.000 g 2-Ethylhexanal und 500 mg Rutheniumnitrosylnitrat eingefüllt. Anschließend wurde der Ansatz bei 180 °C und 2 · 10⁷ Pa (200 bar) Wasserstoffdruck 10 h lang hydriert. Der Umsatz zum gewünschten 2-Ethylhexanol lag bei 100% bei einer Selektivität von 97,2%, jeweils bezogen auf die eingesetzte Menge an 2-Ethylhexanal.

### Beispiel 20

In einem 0,3 l-Rührautoklaven wurden 100 ml Adipinsäuredimethylester an einem Metallnetz und 100 mg Ruthenium(III)acetylacetonat umgesetzt. Der Ansatz wurde 12 h lang bei einem Wasserstoffdruck von 2 · 10⁷ Pa (200 bar) und einer Temperatur von 220 °C gerührt. Die gaschromatographische Analyse des Reaktionsaustrages ergab einen Umsatz von 98% und eine Hexandiol-Ausbeute von 91%, jeweils bezogen auf die eingesetzte Menge an Adipinsäuredimethylester.

## Patentansprüche

1. Verfahren zur Durchführung einer katalytischen Umsetzung einer organischen Verbindung in Gegenwart eines Katalysators, wobei der Katalysator eine auf einem Träger aufgebrachte Rutheniumverbindung umfaßt, **dadurch gekennzeichnet, daß** der Katalysator während der katalytischen Umsetzung der organischen Verbindung durch das Abscheiden einer homogenen Rutheniumverbindung auf dem Träger gebildet wird.

2. Verfahren nach Anspruch 1, wobei die Umsetzung eine Hydrierung, Dehydrierung, Hydrogenolyse, aminierende Hydrierung oder Dehalogenierung ist.

3. Verfahren nach Anspruch 1 oder 2, wobei die organische Verbindung ausgewählt wird aus der Gruppe bestehend aus einer aromatischen Verbindung, in der mindestens eine Hydroxylgruppe an einen aromatischen Kern gebunden ist, einer aromatischen Verbindung, in der mindestens eine Aminogruppe an einen aromatischen Kern gebunden ist, einem Keton, einem Aldehyd, einer Carbonsäure oder einem Derivat davon, einem Polymer, das mindestens eine C-C-Doppelbindung aufweist, einem Polymer, das mindestens eine C=O-Gruppe aufweist, und einem Gemisch aus zwei oder mehr davon.

4. Verfahren nach einem der Ansprüche 1 bis 3, wobei der Träger ausgewählt wird aus der Gruppe bestehend aus einem Metallnetz, Metallring, Steatitkörper; Aktivkohle, Siliciumcarbid, Aluminiumoxid, Siliciumdioxid, Titandioxid, Zirkoniumdioxid, Magnesiumoxid, Zinkoxid und Gemischen aus zwei oder mehr davon, jeweils in Kugel-, Strang- oder Ringform.

5. Verfahren nach einem der vorstehenden Ansprüche, wobei der Träger ein Metallnetz, das Aluminium enthält, ein Metallnetz, auf das eine Palladiummetallschicht in einer Dicke von 0,5 bis 10 nm aufgedampft wurde, oder eine Metallnetz, das Aluminium enthält und auf das eine Palladiummetallschicht in einer Dicke von 0,5 bis 10 nm aufgedampft wurde, ist.

6. Verfahren nach einem der vorstehenden Ansprüche, wobei die homogene Rutheniumverbindung ausgewählt wird aus der Gruppe bestehend aus Nitrosylnitraten, Nitraten, Halogeniden, Carbonaten, Carboxylaten, Chlor-, Nitrido- und Amin-Komplexen, sowie Oxidhydraten des Rutheniums, und einem Gemisch aus zwei oder mehr davon.

7. Verfahren nach einem der vorstehenden Ansprüche, wobei die homogene Rutheniumverbindung zusammen mit der organischen Verbindung in einen Reaktor eingebracht wird.

8. Verfahren nach einem der Ansprüche 1 bis 6, wobei die homogene Rutheniumverbindung vor der Umsetzung in einen Reaktor eingebracht wird.

9. Verfahren nach einem der vorstehenden Ansprüche, wobei die Umsetzung in Gegenwart eines Lösungs- oder Verdünnungsmittels durchgeführt wird.

## Claims

1. A process for carrying out a catalytic reaction of an organic compound in the presence of a catalyst comprising a ruthenium compound applied to a support, wherein the catalyst is formed by deposition of a homogeneous ruthenium compound on the support during the catalytic reaction of the organic compound.

2. A process as claimed in claim 1, wherein the reaction is a hydrogenation, dehydrogenation, hydrogenolysis, aminating hydrogenation or dehalogenation.

3. A process as claimed in claim 1 or 2, wherein the organic compound is selected from the group consisting of aromatic compounds in which at least one hydroxyl group is bound to an aromatic ring, aromatic compounds in which at least one amino group is bound to an aromatic ring, ketones, aldehydes, carboxylic acids and derivatives thereof, polymers containing at least one C-C double bond, polymers containing at least one C=O group and mixtures of two or more thereof.

4. A process as claimed in any of claims 1 to 3, wherein the support is selected from the group consisting of metal meshes, metal rings, steatite bodies, activated carbon, silicon carbide, aluminum oxide, silicon dioxide, titanium dioxide, zirconium dioxide, magnesium oxide, zinc oxide and mixtures of two or more thereof, in each case in spherical, extrudate or ring form.

5. A process as claimed in any of the preceding claims, wherein the support is a metal mesh comprising aluminum, a metal mesh on which a palladium metal layer having a thickness of from 0.5 to 10 nm has been vapor deposited, or a metal mesh which comprises aluminum and on which a palladium metal layer having a thickness of from 0.5 to 10 nm has been vapor deposited.

6. A process as claimed in any of the preceding claims, wherein the homogeneous ruthenium compound is selected from the group consisting of nitrosyl nitrates, nitrates, halides, carbonates, carboxylates, chloro, nitrido and amine complexes and hydrated oxides of ruthenium and mixtures of two or more thereof.

7. A process as claimed in any of the preceding claims, wherein the homogeneous ruthenium compound is introduced together with the organic compound into a reactor.

8. A process as claimed in any of claims 1 to 6, wherein the homogeneous ruthenium compound is introduced into a reactor prior to the reaction.

9. A process as claimed in any of the preceding claims, wherein the reaction is carried out in the presence of a solvent or diluent.

## Revendications

1. Procédé de réalisation d'une réaction catalytique d'un composé organique en présence d'un catalyseur, où le catalyseur comprend un composé du ruthénium appliqué sur un support, **caractérisé en ce que** la formation du catalyseur a lieu, lors de la réaction catalytique du composé organique, par le moyen d'une précipitation d'un composé du ruthénium homogène sur le support.

2. Procédé selon la revendication 1, **caractérisé en ce que** la réaction est une hydrogénation, une déhydrogénation, une hydrogénolyse, une hydrogénation aminée ou une déhalogénation.

3. Procédé selon la revendication 1 ou 2, **caractérisé en ce que** l'on choisit le composé organique dans le groupe formé par un composé aromatique présentant au moins un groupe hydroxyle lié à un noyau aromatique, un composé aromatique présentant au moins un groupe amino lié à un noyau aromatique, une cétone, un aldéhyde, un acide carboxylique ou un de ses dérivés, un polymère possédant au moins une double liaison C=C, un polymère possédant au moins un groupe C=O-, et un mélange de deux ou plusieurs de ces composés.

4. Procédé selon l'une quelconque des revendications 1 à 3, **caractérisé en ce que** l'on choisit le support dans le groupe formé par une grille métallique, un anneau métallique, un corps de stéatite, le charbon actif, le carbure de silicium, l'oxyde d'aluminium, le dioxyde de silicium, le dioxyde de titane, le dioxyde de zirconium, l'oxyde de magnésium, l'oxyde de zinc, et un mélange de deux ou plusieurs de ces supports, chaque fois sous forme sphérique, en forme de corde ou en forme d'anneau.

5. Procédé selon l'une quelconque des revendications précédentes, **caractérisé en ce que** le support est constitué d'une grille métallique contenant de l'aluminium, d'une grille métallique sur laquelle on a déposé sous vide une couche métallique de palladium d'une épaisseur comprise entre 0,5 nm et 10 nm, ou d'une grille métallique contenant de l'aluminium et sur laquelle on a déposé sous vide une couche métallique de palladium d'une épaisseur comprise entre 0,5 nm et 10 nm.

6. Procédé selon l'une quelconque des revendications précédentes, **caractérisé en ce que** l'on choisit le composé du ruthénium homogène dans le groupe formé par les nitrosyl-nitrates, les nitrates, les halogénures, les carbonates, les carboxylates, les complexes de chlore, de nitrure et d'amine, ainsi que les hydrates d'oxyde du ruthénium, et un mélange de deux ou plusieurs de ces composés.

7. Procédé selon l'une quelconque des revendications précédentes, **caractérisé en ce que** l'on introduit le composé du ruthénium homogène et le composé organique conjointement dans le réacteur.

8. Procédé selon l'une quelconque des revendications 1 à 6, **caractérisé en ce que** l'on introduit le composé du ruthénium homogène dans le réacteur avant la réaction.

9. Procédé selon l'une quelconque des revendications précédentes, **caractérisé en ce que** l'on réalise la réaction en présence d'un solvant ou d'un diluant.
